Europäisches Patentamt

European Patent Office

Office européen des brevets

(19)

(11) **EP 0 602 052 B1**

(12) **EUROPÄISCHE PATENTSCHRIFT**

(45) Veröffentlichungstag und Bekanntmachung des Hinweises auf die Patenterteilung:
**13.03.1996 Patentblatt 1996/11**

(51) Int. Cl.⁶: **A61F 2/00**, A61F 2/50,
A61L 27/00, A61B 17/58

(21) Anmeldenummer: **92915062.1**

(22) Anmeldetag: **24.07.1992**

(86) Internationale Anmeldenummer: **PCT/DE92/00597**

(87) Internationale Veröffentlichungsnummer:
**WO 93/03684 (04.03.1993 Gazette 1993/06)**

(54) **EPITHESE UND VORRICHTUNG ZUR LÖSBAREN BEFESTIGUNG DIESER EPITHESE AN EINEM KNOCHEN**

EPITHESIS AND DEVICE FOR REVERSIBLE ATTACHMENT THEREOF TO A BONE

EPITHESE ET DISPOSITIF POUR SA FIXATION AMOVIBLE SUR UN OS

(84) Benannte Vertragsstaaten:
**AT BE CH DE DK ES FR GB IT LI NL SE**

(30) Priorität: **13.08.1991 DE 4126735**

(43) Veröffentlichungstag der Anmeldung:
**22.06.1994 Patentblatt 1994/25**

(73) Patentinhaber: **HORLITZ, Sieglinde**
**D-41541 Dormagen (DE)**

(72) Erfinder: **HORLITZ, Sieglinde**
**D-41541 Dormagen (DE)**

(74) Vertreter: **Bauer, Wulf, Dr.**
**Bayenthalgürtel 15**
**D-50968 Köln (Marienburg) (DE)**

(56) Entgegenhaltungen:
| | |
|---|---|
| EP-A- 0 231 400 | WO-A-83/02047 |
| WO-A-90/11059 | DE-A- 2 455 828 |
| DE-A- 3 844 661 | DE-C- 3 942 674 |
| US-A- 4 416 629 | US-A- 4 676 802 |

**Beschreibung**

Die Erfindung bezieht sich auf eine Epithese und Vorrichtung zu ihrer lösbaren Befestigung nach dem Oberbegriff des Patentanspruchs 1. Eine solche Epithese und Vorrichtung ist z.B. aus der WO 83/02047 bekannt.

Epithesen und Vorrichtungen zu ihrer lösbaren Befestigung sind bekannt beispielsweise aus der US-Fachzeitschrift "The International Journal Of Oral & Maxillofacial Implants" aus 1986. Verwiesen wird hierzu auf die US-Patente 4,330,891 und 4,498,461, sowie auf die WO-Veröffentlichungen WO 90/11059 und WO 83 02047. Dabei wird die Erkenntnis benutzt, daß in Knochensubstanz eingearbeitete Pfosten aus Titan oder einem anderen, geeigneten Werkstoff ohne Bildung einer Zwischenschicht von Knochensubstanz umwachsen werden und dabei so eingebettet werden, daß an ihnen wiederum andere Teile befestigt werden können.

Nach dem Stand der Technik (siehe bespielsweise WO 83/02047) werden in die implantierten Pfosten Schrauben eingedreht, die Köpfe der einzelnen, so eingedrehten Schrauben werden durch eine Drahtstruktur miteinander verbunden. Diese Drahtstruktur befindet sich in einem Abstand von wenigen Millimetern von der Haut und ist damit von außen sichtbar. In einer ersten Ausführung wird die eigentliche Epithese auf die Drahtstruktur aufgeklipst, hierzu hat die Epithese, die größtenteils aus Silikonkautschuk hergestellt ist, in das Silikonmaterial eingebettete und dem Verlauf der Drahtstruktur entsprechend nachgebildete Kunststoffklips, die auf die Drahtstruktur aufgedrückt werden und die Epithese somit an dieser halten. In einer anderen Ausführung hat die Drahtstruktur Permanentmagnete, in die Epithese selbst sind Gegenmagnete an den entsprechenden Orten eingebettet, so daß insgesamt ein magnetischer Halt der Epithese an der Drahtstruktur erreicht wird.

Diese vorbekannte Vorrichtung hat eine Reihe von Nachteilen. So ist es sehr arbeitsaufwendig, die Drahtstruktur herzustellen. Die Drahtstruktur hindert aber auch eine Reinigung der Haut, bzw. erschwert die Reinigung, wenn sie als abnehmbare Drahtstruktur ausgebildet ist. Für den Patienten ist die Drahtstruktur störend, nach Abnahme der Epithese beim Waschen oder Duschen verbleibt eine starre Struktur, die hinderlich ist und als fremd empfunden wird. Darüberhinaus ist auch die Epithese arbeitsaufwendig herzustellen, denn in das Silikonkautschukmaterial müssen die entsprechenden Gegenstücke, also Klips oder Magnete eingebettet werden. Das Kautschukmaterial verbindet sich jedoch weder mit den Magneten noch mit den Klips, so daß hier wiederum spezielle Vorkehrungen getroffen werden müssen. Eine wirklich sichere, dauerhafte Einbettung der Gegenstücke in das Silikonmaterial wird jedoch auf diese Weise nicht erreicht.

Ausgehend von der Vorrichtung der eingangs genannten Art hat sich die Erfindung die Aufgabe gestellt, eine Epithese mit Befestigungsvorrichtung anzugeben und so zu verbessern, daß die Befestigungsvorrichtung deutlich vereinfacht wird, die Herstellung der Befestigungsvorrichtung und damit die Herstellung der Epithese ebenfalls deutlich vereinfacht werden, daß für den Patienten Anbringen und Abnehmen der Epithese vereinfacht ist, wobei ein ausreichend sicherer Halt der Epithese, wie er bei der magnetischen Befestigung nicht immer zu erreichen ist, gewährleistet ist und auf eine Drahtstruktur verzichtet werden kann.

Diese Aufgabe wird gelöst durch die Merkmale des Patentanspruchs 1.

Diese Lösung hat den entscheidenden Vorteil, daß die eigentliche Epithese aus Silikonkunststoff ohne Erfordernis irgendeiner Einbettung eines Klipses oder eines Magneten hergestellt werden kann. Dadurch wird die Herstellung der Epithese vereinfacht, es entfallen auch die Probleme der Verbindung des Silikonkautschukmaterials mit einem Klips oder einem Magneten. Am Patienten entfällt eine Drahtstruktur, vielmehr stehen bei abgenommener Epithese nur mehrere Schrauben mit pilzförmigen Kopfbereichen von dem Hautbereich vor, auf den die Epithese aufgesetzt wird. Die Schrauben stören beim Waschen oder Duschen nicht, die Haut um sie herum kann problemlos gesäubert werden. Insgesamt wird ein sehr sicherer Halt erzielt, der durch Abstimmung des Maßes des Hinterschnitts (Hinterschnittverhältnis) zur Elastizität des verwendeten Silikonkautschuks noch jeweils optimiert werden kann.

Für den Arzt ist die Herstellung der Epithese mit Befestigungsvorrichtung deutlich vereinfacht. Insbesondere vereinfacht sich die Herstellung dann zusätzlich, wenn ein bei Raumtemperatur vulkanisierender Silikonkautschuk verwendet wird.

Als sehr vorteilhaft hat es sich erwiesen, daß die Kopfbereiche an ihrem freien Ende ein Saugloch aufweisen. Das Saugloch ist eine Sackbohrung beispielsweise in Kegel-, Halbkugel- oder Stumpfkegelform. Wenn bei der Herstellung der Epithese die Kopfbereiche der Schrauben in die noch nicht vulkanisierte Silikonkautschukmasse eingebettet werden, werden die Sauglöcher so gefüllt, daß in sie keine Kautschuk masse einlaufen kann. Sie werden beispielsweise mit Wachs ausgefüllt. Nach dem Ausvulkanisieren und Entformen werden die Sauglöcher wieder freigelegt. Sie bewirken später eine zusätzliche Adhäsion zwischen dem Kautschukmaterial der Epithese und den Kopfbereichen, wie sie auch bei Saugnäpfen auftritt.

Vorteilhafterweise sind die Kopfbereiche Drehteile, sie weisen somit keine Kanten oder Ecken auf. Dadurch wird die Herstellung vereinfacht und es treten keine Probleme auf bei der Ausrichtung der Kopfbereiche im für die Herstellung der Epithese verwendeten Modell und der Ausrichtung in den im Knochen verankerten Pfosten.

Vorzugsweise beträgt der Hinterschnitt zumindest zwei zu eins, vorzugsweise vier zu eins und mehr. Das Silikonkautschukmaterial weist eine ausgesprochen hohe Dehnbarkeit auf, so daß Hinterschnitte mit großem Durchmesserverhältnis realisiert werden können, ohne daß beim Abnehmen oder Aufdrucken der Epithese die Gefahr besteht, daß das Kautschukmaterial einreißt. Unter einem Hinterschnittverhältnis von beispielsweise zwei zu eins wird verstanden, daß ausgehend vom freien Ende der Kopfbereich an seiner dicksten Stelle einen Durchmesser hat, der doppelt so groß ist wie der kleinste Durchmesser in einem nachfolgenden Halsbereich.

Eine weitere Vereinfachung bei der Herstellung der Epithese bedeutet die Verwendung von bei Raumtemperatur vulkanisierendem Silikonkautschuk, auf den bereits eingegangen wurde. Ein derartiger Kautschuk wird von der Firma Wacker unter der Bezeichnung "Wakker-Silicon-Kautschuk RTV-M 539" angeboten. Es handelt sich um einen streichbaren, standfesten, kondensationsvernetzenden Silikonkautschuk mit außerordentlichem Ein- und Weiterreißwiderstand und einem weichen, extrem dehnbaren Vulkanisat. Die Konsistenz ist weich-pastös. Das Vulkanisat ist schließlich atoxisch und nicht ermüdend.

Ausführungsbeispiele der Erfindung, die insbesondere auch der Erläuterung des erfindungsgemäßen Verfahrens dienen, werden im folgenden beschrieben. Sie sind nicht einschränkend zu verstehen. Aus ihnen gehen weitere Vorteile und Merkmale der Erfindung hervor. In der Zeichnung zeigen:

Fig. 1 eine schnittbildliche Darstellung (in Form eines Montagebildes) durch einen im Knochengewebe verankerten Pfosten mit Innengewinde, eine Schraube (Saugretentionsknopf) und (darüber) durch eine Epithese,

Fig. 2 ein Schnittbild durch eine Schraube (Retentionsknopf) entsprechend Fig. 1, jedoch in anderer Ausbildung,

Fig. 3 ein Schnittbild durch einen Saugretentionsknopf ähnlich Fig. 1 und

Fig. 4 eine Seitenansicht eines Saugretentionsknopfes.

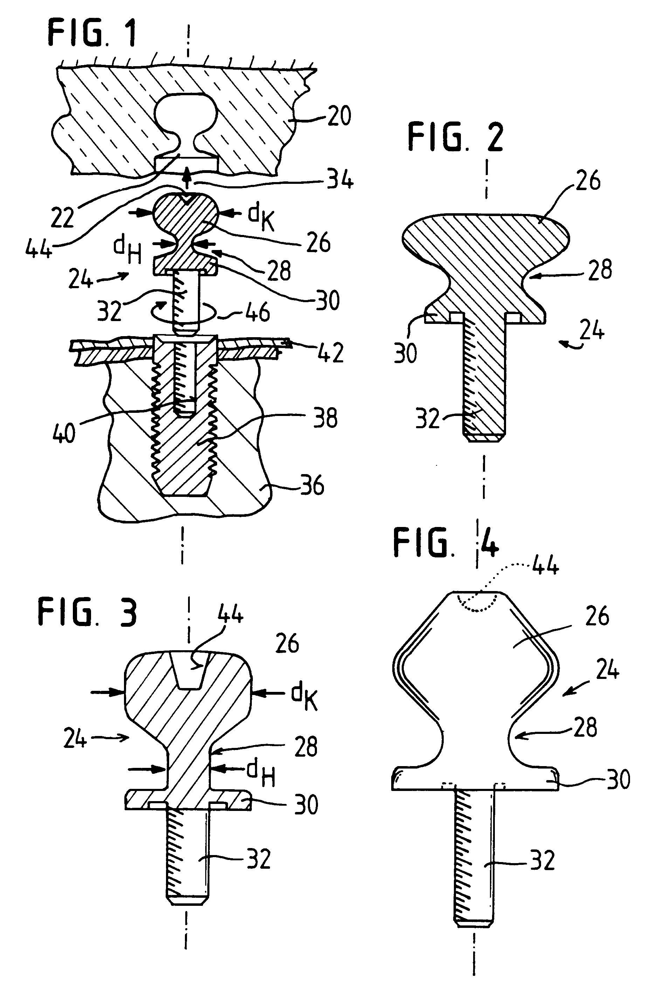

In Fig. 1, das ein Montagebild darstellt, ist oben ein Teil einer Epithese 20 dargestellt. Sie ist aus einem Silikonkautschuk RTV-M 539 der Firma Wacker gefertigt und der Hautfarbe eines Patienten entsprechend eingefärbt. An ihrer in der Fig. 1 nach oben weisenden Oberfläche hat sie eine Hautstruktur, in die einzelne Härchen eingebettet sind. Bei der Epithese handelt es sich beispielsweise um ein Ohr, eine Nase etc.

Die Epithese 20 wird über mehrere Befestigungsvorrichtungen, auf die im folgenden im einzelnen eingegangen wird, gehalten. In Fig. 1 ist lediglich eine Befestigungsvorrichtung gezeigt, es sind jedoch mindestens zwei, vorzugsweise mehrere derartiger Befestigungsvorrichtungen, die vorzugsweise baugleich ausgeführt sind, vorgesehen.

An der Epithese selbst ist eine Ausnehmung 22 auf der Unterseite pro Befestigungsvorrichtung vorgesehen. Sie weist einen Hinterschnitt auf, weitet sich also ausgehend von einem Eingangsbereich nach innen hin auf.

Unterhalb des dargestellten Teilstücks einer Epithese 20 befindet sich eine Schraube 24. Sie setzt sich einstückig zusammen aus einem Kopfbereich 26, einem Halsbereich 28, einem Kragen 30 und einem Gewindebereich 32. Bis auf das Gewinde des Gewindebereichs ist die Schraube 24 ein Drehteil. Die Ausnehmung 22 entspricht formmäßig dem Verlauf von Kopfbereich 26, Halsbereich 28 und Kragen 30. Bei Druck im Sinne des Pfeils 34 schnappen diese Teile 26 bis 30 in die Ausnehmung 22 ein und sichern einen wechselseitigen Halt.

Der Kopfbereich 26 hat einen größten Außendurchmesser $d_K$, Kopfkreisdurchmesser genannt. Der Halsbereich 28 hat einen kleinsten Durchmesser $d_H$, Halsdurchmesser genannt. Beide Durchmesser ins Verhältnis gesetzt werden als Hinterschnittverhältnis bezeichnet, im Ausführungsbeispiel nach Fig. 1 beträgt dieses Hinterschnittverhältnis $d_K/d_H = 3,2$. Das Hinterschnittverhältnis wird angepaßt an die Härte und die Dehnbarkeit des vulkanisierten Silikonkautschuks. Bei härter eingestellten Vulkanisaten wird das Hinterschnittverhältnis geringer gewählt, beispielsweise 2. Bei weich eingestellten Vulkanisaten wird das Hinterschnittverhältnis vergrößert, es beträgt dann beispielsweise 5.

Unterhalb der Schraube 24 schließlich befindet sich in Fig. 1 der implantierte Teil der Befestigungsvorrichtung. In einen Knochen 36 ist eine Ausnehmung eingearbeitet, in diese wiederum ist ein Pfosten 38 mit Innengewinde 40 eingesetzt. Nach außen hin befinden sich eine Zwischenschicht und eine Hautschicht 42 oberhalb des Knochens und seitlich des Pfostens 38, wodurch der Pfosten 38 und insbesondere sein Innengewinde 40 jederzeit von außen frei zugänglich sind. Das Innengewinde 40 ist dem Gewindebereich 32 entsprechend ausgebildet. Der knochenresidente Teil der Befestigungsvorrichtung ist ansich bekannt, hier wird auf den Stand der Technik verwiesen, eine präzise Beschreibung erübrigt sich daher.

Schraube 24 und Pfosten 38 sind aus identischem Material gefertigt, beispielsweise Titan oder ein anderes Metall. Es können jedoch auch Kunststoffe oder keramische Materialien Verwendung finden.

Am freien Ende des Kopfbereichs 26 befindet sich ein Saugloch 44. Es wird beim Abguß der Schraube 24 zur Herstellung der Ausnehmung 22 der Epithese 20 verschlossen, demgemäß hat die Ausnehmung 22 auch keinen dem Saugloch 44 entsprechenden Vorsprung. Dadurch bleibt im Saugloch 44 bei in die Ausnehmung 22 eingesetztem Kopfbereich 26 ein Luftvolumen, in dem sich Luft bei Unterdruck befindet. Hierdurch wird die Haftwirkung unterstützt.

Der Kragen 30 muß nicht notwendigerweise vorhanden sein. Er hat, wie aus Fig. 1 ersichtlich ist, den gleichen Außendurchmesser wie das oberhalb der Hautschicht 42 vorstehende Ende des Pfostens 38, so daß ein sauberer Abschluß an dieser Stelle erzielt wird.

Bei Zusammensetzen wird die Schraube 24 zunächst im Sinne des Pfeiles 46 in das Innengewinde 40 eingeschraubt, bis die Unterseite des Kragens 30 an der Oberseite des Pfostens 38 zur Anlage kommt. Dieses Eindrehen kann per Hand geschehen. Anschließend wird die Epithese 20 aufgedrückt, indem - wie beim Schließen eines Druckknopfes - etwas Druck gegeben wird, bis der Kopfbereich 26 in die zugehörige Ausnehmung 22 einschnappt. Gleiches wird bei den benachbarten Befestigungsbereichen durchgeführt. Dann ist die Epithese befestigt. Sie kann wieder von der Schraube 24 gelöst werden, indem sie nach oben hin abgezogen wird.

Fig. 2 zeigt eine Schraube 24 ohne Saugloch 44 und in Pollerform. Hier ist die Pilzform des Kopfbereiches 26 klar ausgebildet. Der Kopfdurchmesser ist größer als der Außendurchmesser des Kragens 30. Es wird insgesamt eine geringere Bauhöhe erreicht als bei der Schraube nach Fig. 1.

Fig. 3 zeigt ein weiteres Ausführungsbeispiel für eine Schraube 24. Das Hinterschnittverhältnis $d_K$ /$d_H$ beträgt hier etwa 3. Der Kopfbereich 26 hat ein kegelstumpfförmiges Saugloch 44. Der Halsbereich 28 ist länger als bei den bisher besprochenen Ausführungsbeispielen, wodurch größere Abzugskräfte beim Entfernen und ebenso größere Kräfte beim Aufbringen notwendig sind. Der Kragen 30 hat eine sehr geringe Bauhöhe.

Fig. 4 schließlich zeigt ein Ausführungsbeispiel für eine Schraube 24, bei der das freie Ende des Kopfbereichs 26, in dem wiederum ein in diesem Fall halbkugelförmiges Saugloch 44 vorgesehen ist, eine gewisse Zuspitzung bzw. Kegelstumpfform aufweist. Hierdurch soll erreicht werden, daß beim Anlegen der Epithese 20 der Kopfbereich 26 problemfrei die Ausnehmung 22 findet, ohne daß die Mittellinien (und Rotationsachsen) von Ausnehmung 22 und Kopfbereich 26 exakt übereinstimmen müssen. Vielmehr zentriert sich der Kopfbereich 26 im Ausführungsbeispiel nach Fig. 4 durch seine nach oben zugespitzte Form selbst.

**Patentansprüche**

1. Epithese und Befestigungsvorrichtung für die Epithese (20) an einem Knochen (36), wobei die Befestigungsvorrichtung mehrere in den Knochen (36) implantierbare Pfosten (38) mit Innengewinde (40) enthält, die von außen zugänglich sind und in die Schrauben (24) lösbar einsetzbar sind und auch Schrauben aufweist, welche einen nach außen vorstehenden mit einem Hinterschnitt versehenen Kopfbereich (26) haben und der Befestigung der Epithese dienen, dadurch gekennzeichent, daß die Epithese (20) aus weichelastischem Material gefertigt ist und mehrere, sich nach innen hin erweiternde, im weichelastischen Material der Epithese ausgebildete Ausnehmungen (22) hat, und daß die Schrauben (24) einen den Ausnehmungen (22) formmäßig angepaßten Kopfbereich (26), insbesondere einen pilzförmigen Kopfbereich, haben, der somit in die Ausnehmungen (22) druckknopfartig einrasten kann.

2. Epithese mit Befestigungsvorrichtung nach Anspruch 1, dadurch gekennzeichnet, daß die Kopfbereiche (26) an ihrem freien Ende ein Saugloch (44) aufweisen.

3. Epithese mit Befestigungsvorrichtung nach Anspruch 1, dadurch gekennzeichnet, daß die Kopfbereiche (26) Drehteile sind.

4. Epithese mit Befestigungsvorrichtung nach Anspruch 1, dadurch gekennzeichnet, daß der Kopfbereich (26) sich von einem größten Durchmesser $d_K$ hin zu einem Halsbereich (28) verjüngt, dessen kleinster Durchmesser $d_H$ beträgt und daß das Verhältnis $d_K$ zu $d_H$ zumindest zwei, vorzugsweise zumindest drei beträgt.

5. Epithese mit Befestigungsvorrichtung nach Anspruch 1, dadurch gekennzeichnet, daß der Kopfbereich (26) ein abgerundetes Außenprofil aufweist.

6. Epithese mit Befestigungsvorrichtung nach Anspruch 1, dadurch gekennzeichnet, daß der Kopfbereich (26) an seinem freien Ende eine Verjüngung (Fig. 4) aufweist.

7. Epithese mit Befestigungsvorrichtung nach Anspruch 1, dadurch gekennzeichnet, daß die Epithese (20) aus einem bei Raumtemperatur vulkanisierenden Silikonkautschuk mit weichpastöser Konsistenz und hoher Dehnbarkeit gefertigt ist.

**Claims**

1. Epithesis and fixing device for the epithesis (20) to a bone (36), wherein the fixing device comprises a number of fixtures (38) with internal thread, which fixtures (38) are implantable in the bone (36), the fixtures being accessible form outside and screws (24) are removably insertable into them, and wherein the fixing device further comprises screws having a head (26) protruding outwards, having an undercut, and serving to attach the epithesis,
characterized in that the epithesis is manufactured from soft-elastic material, in that an number of recesses (22) are provided in the soft-elastic material of the epithesis, which recesses (22) widen inwards, and in that the screws (24) exhibit a head (26) matched in form to the recesses (22), especially exhibit a mushroom-shaped head, which can engage in a press-stud-like fashion into the recesses (22).

2. Epithesis with fixing device according to Claim 1, characterized in that the heads (26) have a suction hole (44) on their free ends.

3. Epithesis with fixing device according to Claim 1, characterized in that the heads (26) are turned parts.

4. Epithesis with fixing device according to Claim 1, characterized in that the head (26) tapers from a greatest diameter $d_K$ to a neck (28), the smallest diameter of wich is $d_H$, and that the ratio of $d_K$ to $d_H$ is not less than 2, and preferably not less than 3.

5. Epithesis with fixing device according to Claim 1, characterized in that the head (26) has a rounded external profile.

6. Epithesis with fixing device according to Claim 1, characterized in that the head (26) has a taper (Fig. 4) at its free.

7. Epithesis with fixing device according to Claim 1, charcterized in that the epithesis (20) is manufactured from a silicone rubber which vulcanizes at room temperature, of soft pasty consistency and of high extensibility.

**Revendications**

1. Epithèse ainsi que dispositif de fixation de ladite épithèse (20) sur un os (36), le dispositif de fixation comportant plusieurs potelets (38) pourvus d'un filet de vis intérieur (40) qui peuvent être implantés dans l'os (36) et qui sont accessibles par dehors et dans lesquels des vis (24) peuvent être introduites de manière amovible, ledit dispositif comportant en sus des vis qui présentent une section de tête (26) faisant saillie vers l'extérieur et pourvue d'une contre-dépouille, et qui servent au-delà de ce fait à fixer l'épithèse, caractérisés en ce que l'épithèse (20) est fabriquée en une matière souple et élastique et qu'elle présente plusieurs évidements (22) s'élargisant vers l'intérieur et réalisés en cette même matière souple et élastique que l'épithèse, et que les vis (24) présentent une section de tête (26) adaptée en forme aux évidements (22), et en particulier une section de tête en forme de champignon qui peut par conséquent être introduite jusqu'à encliquetage - à savoir selon le principe d'un bouton-pression - dans les évidements (22).

2. Epithèse avec dispositif de fixation selon la revendication 1, caractérisée en ce que les sections de tête (26) présentent un trou d'aspiration (44) sur leur extrémité libre.

3. Epithèse avec dispositif de fixation selon la revendication 1, caractérisée en ce que les sections de tête (26) sont des pièces tournées.

4. Epithèse avec dispositif de fixation selon la revendication 1, caractérisée en ce que la section de tête (26) se rétrécit d'un diamètre maximal $d_K$ en direction d'une section de col (28) dont le diamètre le plus petit est de $d_H$, et que le rapport de $d_K$ à $d_H$ est du moins deux, et de préférence du moins trois.

5. Epithèse avec dispositif de fixation selon la revendication 1, caractérisée en ce que la section de tête (26) présente un profil extérieur arrondi.

6. Epithèse avec dispositif de fixation selon la revendication 1, caractérisée en ce que la section de tête (26) présente un rétrécissement (figure 4) sur son extrémité libre.

7. Epithèse avec dispositif de fixation selon la revendication 1, caractérisée en ce que l'épithèse (20) est fabriquée en un caoutchouc silicone vulcanisant à la température ambiante, qui présente une consistance souple et pâteuse ainsi qu'une éxtensibilité élevée.

# FIG. 1

# FIG. 2

# FIG. 3

# FIG. 4